# EUROPEAN PATENT SPECIFICATION

(11) **EP 0 945 062 B1**
(45) Date of publication and mention of the grant of the patent: **15.01.2003**
(21) Application number: 99105950.2
(22) Date of filing: 24.03.1999
(51) Int. Cl.: A01M 1/20, A61L 9/03

(54) **Vaporizer for insecticides or perfumes in liquid formulations, with electric heating element fixed to the rotatable plug**
Verdunstungsvorrichtung für Insektizide oder Parfüme in Flüssigformulierungen, mit elektrischen Heizelement das auf dem rotierenden Stecker befestigt wird
Vaporisateur pour insecticide ou parfum liquides, avec élément électrique de chauffage fixé à la prise électrique rotative

(30) Priority: 26.03.1998 IT MI980634
(43) Date of publication of application: 29.09.1999
(73) Proprietor: ZOBELE HOLDING S.P.A., 38100 TRENTO (IT)
(72) Inventor: ZOBELE, Franco, 38100 Trento (IT)
(74) Representative: Faggioni, Marco, Dr. Ing.

(56) References cited:
- WO-A-97/13539
- DE-A- 4 131 613

## Description

The present invention relates to a vaporizer for insecticides or perfumes in liquid formulation, of the type which is provided with a rotatable plug - so as to allow the apparatus to be used both with sockets having horizontal holes and with sockets having vertical holes - and wherein the electric heating device is fixed to the plug and rotates together therewith.

This type of vaporizer was introduced fairly recently onto the market and has proved to be very effective both from the point of view of simplification of the structure of the vaporizer and from the point of view of duration and safety. With this type of design, in fact, there is no need for an electrical connection between two mutually rotating parts - namely the plug and the heating device - as occurs in the known prior art in which the heating device is fixed to the main body of the vaporizer, i.e. the body which forms the housing for the container holding the liquid formulation and the associated wick. An example of the abovementioned technology is disclosed in the patent DE-4,131,613.

The elimination of the flexible electrical connections between the abovementioned two mutually rotating parts therefore makes it possible to simplify the manufactoring method of the vaporizer, facilitating automation thereof, since the two parts of the vaporizer may be assembled separately and then joined together without particular difficulty or the need for manual operations, it being possible to lock perfectly in position the individual components of the two parts already during separate assembly steps. Moreover, owing to the elimination of said flexible connections, a possible easy source of faults and electrical dispersion may be eliminated, thus increasing the safety of the vaporizer.

However, it must be pointed out that the use of electric heating elements fixed to the plug, in contrast to the advantages mentioned above, has nevertheless resulted in some not insignificant drawbacks in the vaporizers. In fact, in vaporizers of the former type, in which the heating device is permanently fixed in the housing of the container holding the liquid formulation, the heating device may be positioned in the most suitable manner with respect to the wick for the liquid formulation and moreover may be provided with suitable means for distribution of the heat, so as to ensure a desirable constant and homogeneous flow of heat around the wick itself, also when, as it happens in most cases, there is only one heating device which is therefore arranged asymmetrically with respect to the wick. This possibility of associating, with the heating device, ceramic or metallic means for distributing the heat around the wick obviously is no longer possible when the heating device is fixed to the rotatable plug. Moreover, the actual correct positioning of the heating device is problematic owing to the fact that the heating device takes up different spatial positions in relation to the wick, when the plug is horizontal or vertical.

In vaporizers of the known type which use a single heating device fixed to the rotatable plug, there are therefore two main drawbacks: namely that of a nonuniform heat distribution around the wick and that of heat distribution which is also substantially different depending on whether the vaporizer is used with the plug in the horizontal or vertical position.

A first object of the present invention is therefore that of providing a vaporizer, of the type with an electric heating device fixed to the rotatable plug, which overcomes the drawbacks described above and which therefore is able to achieve a substantially homogeneous distribution of the heat around the wick, despite the fact that the heating device cannot be mechanically associated with heat distribution means.

Another object of the invention is that of providing a vaporizer of the type described above which is able to achieve constant distribution of the heat around the wick irrespective of the horizontal or vertical position assumed by the rotatable plug.

These objects are achieved, in accordance with the present invention, by means of a vaporizer for insecticides or perfumes in liquid formulations, comprising a housing for supporting the liquid container and confining the associated wick, and a plug body to which an electric heating device is fixed, said plug body being rotatable between at least one vertical plug position and at least one horizontal plug position, characterized in that said housing comprises a cylindrical element having a cross-section partially surrounding said wick, the longitudinal opening of said cylindrical element being directed towards said heating device.

According to an additional feature of the invention, the spatial arrangements which the heating device assumes in said first and second plug positions are symmetrical with respect to a plane passing through the axis of rotation of the plug and parallel to and preferably coinciding with the axis of said wick.

Further characteristic features and advantages of the vaporizer according to the present invention will emerge, anyway, more clearly from the detailed description which follows, provided with reference to the accompanying drawings, in which:
Fig. 1 is an exploded side elevation view of a vaporizer for liquid formulations according to the present invention, provided with a rotatable plug equipped with a first embodiment of the heating device;
Fig. 2 is a front elevation exploded view of the vaporizer according to Fig. 1, with the rotatable plug removed;
Fig. 3 is an exploded plan view of the vaporizer according to Fig. 1 with the top part of the housing removed;
Figs. 4 and 4B are side views, in two positions which are mutually rotated by 90°, of the rotatable plug equipped with a second embodiment of the heating device;
Fig. 5A is a plan view, from underneath and on a larger scale, of the heating device according to Figs. 4;
Fig. 5B is a sectioned view of the heating device along the line B-B of Fig. 5A;
Figs. 6A and 6B are side views, in two positions mutually rotated by 90°, of the rotatable plug equipped with a third embodiment of the heating device; and
Fig. 7 is a rear plan view of the plug according to Fig. 6.

The vaporizer according to the present invention comprises a housing 1 for the container holding the liquid formulation, consisting of two half-shells which can be fitted together by pressure.

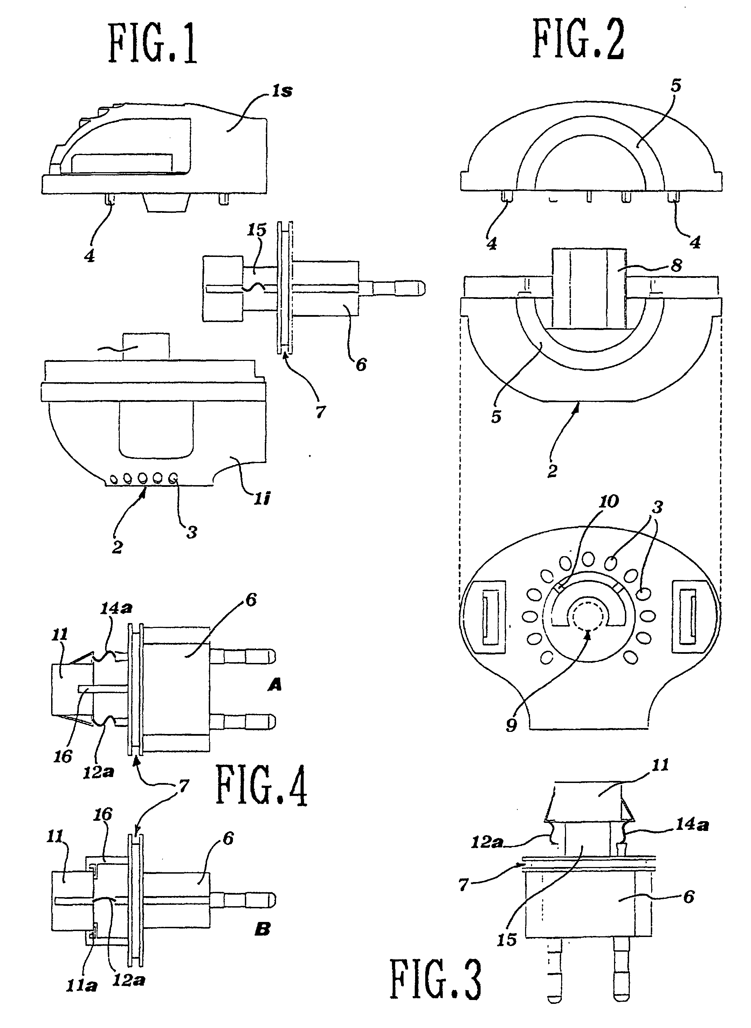

As can be seen in Figures 1 to 3, the bottom half-shell 1i is provided, on its base, with a central opening 2 into which the neck of the container holding the liquid formulation (not shown) is screwed and with air inlet holes 3 arranged in a ring around the opening 2. The top half-shell 1s has, on its bottom edge, a series of projections 4 which are able to engage with a corresponding ribbing formed on the inner side of the top edge of the half-shell li so as to define the exact position for fitting together the two half-shells. Laterally (Fig. 2), both the half-shells 1i and 1s have a semi-circular opening provided with an annular rib 5 which forms, once the two half-shells have been joined together, the support for a rotatable plug body 6. The plug body 6 is provided for this purpose with a disk-shaped base having a peripheral annular groove 7 intended to be engaged with the ribs 5. Finally the said parts may be premanently connected together by means of snap-engagement or screw-type closing devices. The structure described hitherto is entirely conventional and it is therefore not required to provide further details, which in any case are widely known to persons skilled in the art.

As already stated in the introductory part, the vaporizer according to the present invention is of the type in which the electric heating device is permanently fixed to the rotatable plug body and therefore rotates together therewith, when the plug is rotated according to the type of socket available (vertical or horizontal). It is therefore not possible, in this type of vaporizer, to provide those heat distribution means - normally made of ceramic or metal materials - which in the more conventional vaporizers surround both the heating device and the wick housing channel, so as to distribute the heat around the wick as uniformly as possible also when only one heating device is present.

In order to overcome this problem, according to the present invention, it is envisaged that inside the bottom half-shell li of the vaporizer, and precisely in the region of and around the wick housing channel, a cylindrical element 8 with a C-shaped cross-section is provided, said element being coaxial with the wick and extending longitudinally so as to cover the entire length of the wick 9 (shown in broken lines in Fig. 3) which projects from the neck of the container holding the liquid formulation. The cylindrical element 8 is integrally formed with the bottom half-shell 1i or is manufactured separately and forcedly fitted onto the base of the bottom half-shell 1i by means of suitable support brackets 10 projecting from the half-shell or with any other suitable fixing means known per se, the longitudinal opening thereof being arranged so as to face the heating device associated with the rear part of the rotatable plug 6. In the embodiment of the vaporizer according to the present invention shown in the drawings, the cylindrical C-shaped element has a circular cross-section; this definition, however, is understood as comprising any type of cross-section with one quadrant open and the other three closed, for example a U-shaped cross-section, or a square cross-section or a rectangular cross-section, which are devoid of one side, and the like.

When the C-shaped element is formed as one piece with the bottom half-shell and therefore is made of the same material as the latter, the heating device will be located at a certain distance from the C-shaped element, so as to avoid problems arising from localized overheating of the plastic material of the vaporizer which is not suitable for withstanding the high temperatures reached by the heating device. When, on the other hand, the C-shaped element is manufactured separately and then fixed onto the bottom half-shell, it may be advantageously made of plastic material with a higher heat resistance (for example phenylene polysulphide - FPS) or, partly or entirely, of metal. The greater cost of this last type of design is in this case offset by the fact that the heating device may be arranged in the close vicinity of or even in contact with the C-shaped element, and the latter may be shaped so as to match more closely the shape of the heating device, so as to obtain a greater heating efficiency of the wick.

With this particular design, inside the air gap which forms between the cylindrical element 8 and the wick 9 a strong upward air current is produced, owing to the chimney effect, resulting in a rapid and effective homogenization, around the whole of the wick 9, of the heat emitted by the heating device.

According to an additional feature of the present invention, the heating device associated with the rotatable plug has a geometrical configuration and a location such that the spatial arrangements which this device assumes in the horizontal and vertical positions of the plug 6 are perfectly symmetrical with respect to a plane passing through the axis of the plug 6 and parallel to the axis of the wick 9 (the path of which in the plane of the drawing is represented by a dot-dash line in Fig. 3); preferably the axis of the wick 9 lies in the aforementioned plane. In this way, the heating device, in the two, vertical and horizontal, positions which the plug 6 is able to assume (or four positions in the case of an endlessly rotatable plug), has identical or symmetrical positions with respect to the wick 9, to which it therefore supplies an absolutely constant flow of heat, irrespective of the position of the plug 6, thus achieving the second object of the invention.

Figs. 1 to 5 show two embodiments of the heating device in which this device consists of a cylindrical ceramic body 11 containing a posistor PTC and the associated contact plates. More precisely, the bottom of the body 11 seats a flat plate, the contact 12a of which protrudes laterally from a side slit in the body 11 so as to be connected to the pins of the plug 6. The PTC element 13 is arranged above the flat plate 12 and above it the upper plate which is also provided with a contact 14a which protrudes from a slit situated on the opposite side of the cylindrical body 11 with respect to the outlet slit for the contact 12a.

The upper plate 14 has on its periphery four elastic tongues 14b, integral with said plate 14 and designed to engage elastically against the internal walls of the cylindrical body 11 when the plate 14 is pressed inside the body 11, thus securely fixing the assembly consisting of the PTC element 13 and its contact plates 12 and 14. To ensure a reliable electrical contact, also in the event of knocks or vibrations, the plate 14 has centrally a lowered tongue 14c which is cut out from the same plate 14 and is apt to remain elastically pressed against the upper surface of the PTC 13.

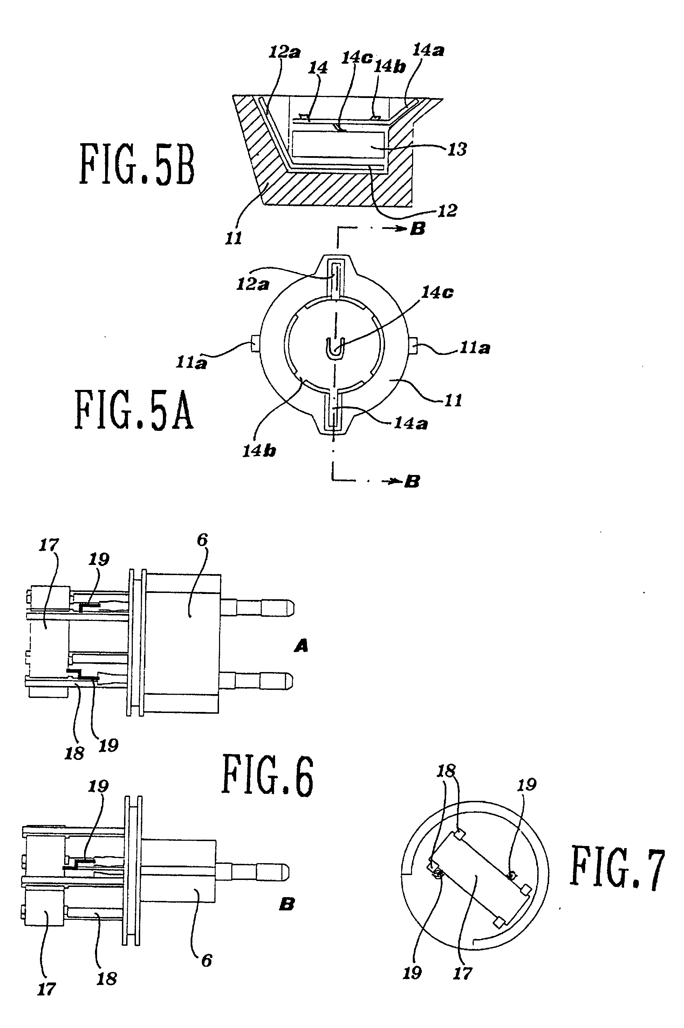

The heating device 11 described hitherto may be fixed to the plug 6 in different ways. In a first embodiment, shown in Figs. 1 and 3, from the disk-shaped base of the plug 7 a plastic hub 15 projects centrally, said hub having an external diameter which is the same as the internal diameter of the ceramic body 11. The latter is then fitted onto the hub 15 and fixed to it with a suitable adhesive. In a second embodiment, shown in Figs. 4 and 5, the cylindrical body has instead two lateral lugs 11a which receive, elastically engaged with them, corresponding plastic brackets 16 integrally formed with the disk-shaped base of the plug 6 and suitable for keeping the heating device 11 in a steady position.

In both cases, the axis of the cylindrical heating device 11 coincides with the axis of the plug 6 such that, when the latter is rotated from the horizontal position to the vertical position, the heating device 6 does not change its spatial position with respect to the wick 9 and therefore there is no variation in the flow of heat from the heating device 11 to the wick.

Figures 6 and 7 show a third embodiment of the heating device, consisting in this case of a cylindrical electric resistance cemented inside a block 17 of ceramic material having the shape of a right-angled square-base parallelepiped. The ceramic block 17 is held in position by means of more plastic brackets 18 integrally formed with the disk-shaped base of the plug 6 and apt to flex elastically so as to hold, by means of an engagement, the block 17 in position. The electric terminals of the electric resistance cemented inside the block 17 may be in the form of flexible electric leads and in this case are manually welded to the pins of the plug 6. Preferably, however, these terminals consist of metal elements 19 which are shaped so as to be sufficiently rigid and have their free end tapered such that they can be easily inserted into the holes in the pins of the plug 6; with this type of terminals it is in fact possible to perform easily assembly of the heating device 6 on the plug 6 in a completely automatic manner.

As is obvious from the plan view of Fig. 7, the longitudinal axis of the heating device 17 lies in a plane parallel to the plane of the disk-shaped base of the plug 6 and inclined at 45° with respect to the plane which contains the two pins of the plug 6, in contrast to the situation in vaporizers of the known type, in which the longitudinal axis of the heating device lies in the same plane which contains the pins of the plug. In this way, in the horizontal and vertical positions of the plug 6, the heating device 17 assumes spatial arrangements which are perfectly symmetrical with respect to the aforementioned plane of symmetry, such that the quantity of heat which this device transmits to the wick 9 is entirely the same in all the horizontal or vertical positions of the plug.

## Claims

1. Vaporizer for insecticides or perfumes in liquid formulations, comprising a housing (1) for supporting the liquid container and confining the associated wick (9) , and a plug body (6) to which an electric heating device (11, 17) is fixed, said plug body (6) being rotatable between at least one vertical plug position and at least one horizontal plug position, **characterized in that** said housing (1) comprises a cylindrical element (8) having a cross-section partially surrounding said wick, the longitudinal opening of said cylindrical element (8) being directed towards said heating devices (11, 17).

2. Vaporizer as claimed in claim 1, wherein said cylindrical element (8) has a C-shaped cross-section.

3. Vaporizer as claimed in Claim 2, wherein said C-shaped cylindrical element (8) is integrally formed with said housing (1).

4. Vaporizer as claimed in Claim 2, wherein said C-shaped cylindrical element (8) is formed separately from said housing (1) and is fixed thereto in an engaging fit, by means of support brackets (10).

5. Vaporizer as claimed in Claim 4, wherein said C-shaped cylindrical element (8) is made from a plastic material with a high heat resistance or, at least partly, from a metallic material.

6. Vaporizer as claimed in Claim 5, wherein said C-shaped cylindrical element (8) is made from metallic material at least in the region near to said heating device (11, 17) and has a shape such as to match the shape of said heating device.

7. Vaporizer as claimed in Claims 1 to 6, wherein the spatial arrangements which the heating device (11, 17) assumes in said first and second plug positions are identical, or symmetrical with respect to a plane passing through the axis of the plug (6) and parallel to said wick (9).

8. Vaporizer as claimed in Claim 7, wherein said plane of symmetry passes through the axis of said wick (9).

9. Vaporizer as claimed in Claims 1 to 8, wherein said heating device consists of a hollow cylindrical body (11) of ceramic material fixed in a coaxial manner with respect to said plug body (6).

10. Vaporizer as claimed in Claim 9, wherein said cylindrical body (11) contains a first flat contact plate (12), a PTC element (13), a second contact plate (14) provided with elastic means (14c) for realizing an electrical contact with the PTC and with elastic means (14b) for gripping the internal walls of said cylindrical body (11).

11. Vaporizer as claimed in Claim 10, wherein said elastic electrical contact means (14c) consist of a central tongue which is cut out from said contact plate (14) and folded back toward the PTC (13).

12. Vaporizer as claimed in Claim 10, wherein said elastic gripping means (14b) consist of peripheral tongues integrally formed with said contact plate (14).

13. Vaporizer as claimed in Claim 9, wherein said cylindrical body (11) is fixed by means of engagement or by means of adhesives onto a coaxial hub (15) projecting from said rotatable plug body (6).

14. Vaporizer as claimed in Claim 9, wherein said cylindrical body (11) is provided with lateral lugs (11a) for elastic fixing, by means of engagement, to lateral brackets (16) projecting from said rotatable plug body (6).

15. Vaporizer as claimed in Claims 1 to 8, wherein said heating device consists of a parallelepiped block (17) made of ceramic material and containing an electric resistance cemented therein, said block (17) being fixed to said plug body (6) with its longitudinal axis arranged in a plane parallel to the disk-shaped base of the plug and inclined at 45° with respect to the plane containing the pins of the plug.

16. Vaporizer as claimed in Claim 15, wherein said heating device is provided with electric terminals consisting of metallic elements (19) shaped so as to have a sufficient rigidity and to have their free end tapered for insertion into the pins of the plug (6).

## Patentansprüche

1. Verdunstungsvorrichtung für Insektizide oder Parfüme in Flüssigformulierungen, umfassend ein Gehäuse (1) zum Halten des Flüssigbehälters und Begrenzen des assoziierten Dochts (9), und einen Steckkörper (6), an den ein elektrisches Heizgerät (11, 17) befestigt ist, wobei der Steckkörper (6) drehbar zwischen wenigstens einer vertikalen Steckerposition und wenigstens einer horizontalen Steckerposition ist, **dadurch gekennzeichnet, daß** das Gehäuse (1) ein zylindrisches Element (8) mit einem Querschnitt aufweist, der teilweise den Docht umgibt, wobei die longitudinale Öffnung des zylindrischen Elements (8) auf das Heizgerät (11, 17) gerichtet ist.

2. Verdunstungsvorrichtung nach Anspruch 1, **dadurch gekennzeichnet, daß** das zylindrische Element (8) einen C-förmigen Querschnitt aufweist.

3. Verdunstungsvorrichtung nach Anspruch 2, **dadurch gekennzeichnet, daß** das C-förmige, zylindrische Element (8) integral mit dem Gehäuse (1) gebildet ist.

4. Verdunstungsvorrichtung nach Anspruch 2, **dadurch gekennzeichnet, daß** das C-förmige, zylindrische Element (8) separat von dem Gehäuse (1) gebildet ist und daran in einem ineinandergreifenden Einbau mittels Halteklammern (10) befestigt ist.

5. Verdunstungsvorrichtung nach Anspruch 4, **dadurch gekennzeichnet, daß** das C-förmige, zylindrische Element (8) hergestellt ist aus einem Kunststoffmaterial mit einer hohen Wärmeresistenz oder, zumindest teilweise, aus einem metallischen Material.

6. Verdunstungsvorrichtung nach Anspruch 5, **dadurch gekennzeichnet, daß** das C-förmige, zylindrische Element (8) hergestellt ist aus metallischem Material wenigstens in dem Bereich in der Nähe des Heizgeräts (11, 17) und eine Form aufweist, um mit der Form des Heizgeräts zusammenzupassen.

7. Verdunstungsvorrichtung nach einem der Ansprüche 1 bis 6, **dadurch gekennzeichnet, daß** die räumlichen Anordnungen, welche das Heizgerät (11, 17) in der ersten und zweiten Steckerposition annimmt, identisch sind, oder symmetrisch in Bezug auf eine Ebene, die durch die Achse des Steckers (6) und parallel zu dem Docht (9) führt.

8. Verdunstungsvorrichtung nach Anspruch 7, **dadurch gekennzeichnet, daß** die Symmetrieebene durch die Achse des Dochts (9) führt.

9. Verdunstungsvorrichtung nach einem der Ansprüche 1 bis 8, **dadurch gekennzeichnet, daß** das Heizgerät aus einem hohlen, zylindrischen Körper (11) aus keramischen Material besteht, welcher auf eine koaxiale Weise in Bezug auf den Steckkörper (6) befestigt ist.

10. Verdunstungsvorrichtung nach Anspruch 9, **dadurch gekennzeichnet, daß** der zylindrische Körper (11) enthält eine erste flache Kontaktplatte (12), ein PTC-Element (13), eine zweite. Kontaktplatte (14), bereitgestellt mit einem elastischen Mittel (14c) zum Realisieren eines elektrischen Kontakts mit dem PTC und mit einem elastischen Mittel (14b) zum Ergreifen der Innenwände des zylindrischen Körpers (11).

11. Verdunstungsvorrichtung nach Anspruch 10, **dadurch gekennzeichnet, daß** die elastischen, elektrischen Kontaktmittel (14c) aus einer Mittelzunge bestehen, welche ausgeschnitten ist aus der Kontaktplatte (14) und zurückgefaltet auf die PTC (13).

12. Verdunstungsvorrichtung nach Anspruch 10, **dadurch gekennzeichnet, daß** das elastische Ergreifungsmittel (14b) aus peripheren Zungen besteht, die integral mit der Kontaktplatte (14) gebildet sind.

13. Verdunstungsvorrichtung nach Anspruch 9, **dadurch gekennzeichnet, daß** der zylindrische Körper (11) befestigt ist mittels eines Ineinandergreifens oder mittels von Klebstoffen auf einer koaxialen Nabe (15), die von dem drehbaren Steckkörper (6) hervorragt.

14. Verdunstungsvorrichtung nach Anspruch 9, **dadurch gekennzeichnet, daß** der zylindrische Körper (11) versehen ist mit seitlichen Ansätzen (11a) zum elastischen Fixieren, mittels eines Ineinandergreifens, an seitliche Klammern (16), welche von dem drehbaren Steckkörper (6) hervorragen.

15. Verdunstungsvorrichtung nach einem der Ansprüche 1 bis 8, **dadurch gekennzeichnet, daß** das Heizgerät aus einem parallelepipeden Block (17) besteht, hergestellt aus keramischem Material und enthaltend einen elektrischen Widerstand, der darin eingeklebt ist, wobei der Block (17) an den Steckkörper (6) mit seiner longitudinalen Achse in einer planparallelen Anordnung zu der scheibenförmigen Basis des Steckers befestigt und geneigt um 45° in Bezug auf die Ebene, welche die Stifte des Steckers enthält, ist.

16. Verdunstungsvorrichtung nach Anspruch 15, **dadurch gekennzeichnet, daß** das Heizgerät versehen ist mit elektrischen Anschlüssen, bestehend aus metallischen Elementen (19), die so geformt sind, um eine ausreichende Steifheit aufzuweisen und um deren freies Ende kegelförmig zum Einsatz in die Stifte des Steckers (6) zu haben.

## Revendications

1. Vaporisateur pour insecticides ou parfums en formulations liquides, comportant un boîtier (1) destiné à supporter le récipient à liquide et renfermant la mèche associée (9), et un corps de fiche (6) auquel un dispositif chauffant électrique (11, 17) est fixé, ledit corps de fiche (6) pouvant être tourné entre au moins une position de fiche verticale et au moins une position de fiche horizontale, **caractérisé en ce que** ledit boîtier (1) comporte un élément cylindrique (8) ayant une section transversale entourant partiellement ladite mèche, l'ouverture longitudinale dudit élément cylindrique (8) étant tournée vers ledit dispositif chauffant (11, 17).

2. Vaporisateur selon la revendication 1, dans lequel ledit élément cylindrique (8) a une section transversale en forme de C.

3. Vaporisateur selon la revendication 2, dans lequel ledit élément cylindrique (8) en forme de C est formé d'une seule pièce avec ledit boîtier (1).

4. Vaporisateur selon la revendication 2, dans lequel ledit élément cylindrique (8) en forme de C est formé séparément dudit boîtier (1) et est fixé à celui-ci en prise d'enclenchement, au moyen de pattes (10) de support.

5. Vaporisateur selon la revendication 4, dans lequel ledit élément cylindrique (8) en forme de C est réalisé en une matière plastique à haute résistance à la chaleur ou, au moins en partie, en une matière métallique.

6. Vaporisateur selon la revendication 5, dans lequel ledit élément cylindrique (8) en forme de C est formé d'une matière métallique au moins dans la région proche dudit dispositif chauffant (11, 17) et a une forme telle qu'elle est adaptée à la forme dudit dispositif chauffant.

7. Vaporisateur selon les revendications 1 à 6, dans lequel les dispositions dans l'espace que le dispositif chauffant (11, 17) prend dans lesdites première et seconde positions de fiche sont identiques, ou symétriques par rapport à un plan passant par l'axe de la fiche (6) et parallèle à ladite mèche (9).

8. Vaporisateur selon la revendication 7, dans lequel ledit plan de symétrie passe par l'axe de ladite mèche (9).

9. Vaporisateur selon les revendications 1 à 8, dans lequel ledit dispositif chauffant consiste en un corps cylindrique creux (11) en matière céramique fixé coaxialement par rapport audit corps de fiche (6).

10. Vaporisateur selon la revendication 9, dans lequel ledit corps cylindrique (11) contient une première lame plate (12) de contact, un élément CTP (13), une seconde lame (14) de contact pourvue d'un moyen élastique (14c) pour réaliser un contact électrique avec l'élément CTP et d'un moyen élastique (14b) pour réaliser une prise sur les parois intérieures dudit corps cylindrique (11).

11. Vaporisateur selon la revendication 10, dans lequel ledit moyen élastique (14c) de contact électrique consiste en une languette centrale qui est découpée dans ladite lame de contact (14) et repliée en arrière vers l'élément CTP (13).

12. Vaporisateur selon la revendication 10, dans lequel ledit moyen élastique (14b) de prise consiste en languettes périphériques formées d'une seule pièce avec ladite lame de contact (&4).

13. Vaporisateur selon la revendication 9, dans lequel ledit corps cylindrique (11) est fixé au moyen d'un enclenchement ou au moyen d'adhésifs dans un moyeu coaxial (15) faisant saillie dudit corps de fiche tournant (6).

14. Vaporisateur selon la revendication 9, dans lequel ledit corps cylindrique (11) est pourvu d'ergots latéraux (11a) pour une fixation élastique, au moyen d'un enclenchement, à des pattes latérales (16) faisant saillie dudit corps de fiche (6) pouvant tourner.

15. Vaporisateur selon les revendications 1 à 8, dans lequel ledit dispositif chauffant consiste en un bloc parallélépipédique (17) formé d'une matière céramique et contenant une résistance électrique collée à l'intérieur de ce bloc, ledit bloc (17) étant fixé audit corps de fiche (6) de façon que son axe longitudinal soit agencé dans un plan parallèle à la base en forme de disque de la fiche et incliné à 45° par rapport au plan contenant les broches de la fiche.

16. Vaporisateur selon la revendication 15, dans lequel ledit dispositif chauffant est pourvu de bornes électriques constituées d'éléments métalliques (19) configurés de façon à avoir une rigidité suffisante et à avoir leurs extrémités libres effilées pour être introduites dans les broches de la fiche (6).
